# EUROPEAN PATENT APPLICATION

(11) **EP 4 790 411 A1**
(43) Date of publication of application: **12.08.2026**
(21) Application number: 25156276.5
(22) Date of filing: 06.02.2025
(51) Int. Cl.: G01N 33/02, G06V 20/68

(54) **METHOD AND SYSTEM FOR ANALYZING QUALITY OF A FOOD ITEM**

(71) Applicant: Nagravision Sàrl, 1033 Cheseaux-sur-Lausanne (CH)
(72) Inventor: SAHOO, Bibhudananda, 560048 Bangalore (IN); JOSEPH, Allwin, 560048 Bangalore (IN); RAVICHANDRAN, Seenuvasan, 560048 Bangalore (IN); REDDY, Gurram Sandeep, 560048 Bangoalore (IN); SAHOO, Gaurav Kumar, 560048 Bangalore (IN); P, Ambika, 560048 Bangalore (IN)
(74) Representative: Novagraaf International SA

(57) **Abstract**

A method and system of analyzing a food item comprising:
- receiving, from a user device, sensor data relating to the food item including image data (ID) and measurement data (MD) including odor data;
- determining a food class (FC) of the food item from a plurality of food classes from the image data and by using a first trained machine learning model, MLM,
- determining a food quality indicator (FQI) of the food item based on the measurement data and the determined food class, and
- transmitting the food class and the food quality indicator to the user device for presentation to the user.

## Description

### TECHNICAL FIELD

The present disclosure relates to a method and system for analyzing quality of a food item.

### BACKGROUND

Food security has become a global concern nowadays. A farmer harvests the crop, but by the time it reaches the consumer, the condition of the crop is either damaged during transport or altered in various ways to maintain its freshness.

Food adulteration is an increasing practice in the food industry, more prevalent in regions of the world with weak regulations, which involves the addition, removal, or alteration of food components and often the use of chemicals, artificial substances, or other non-edible materials. It is aimed at reducing production costs, facilitating transportation over long distances extending shelf life, or enhance the appearance of food.

The application of chemicals and other harmful elements to vegetables, fruits, meat or any agricultural products may significantly impact may have serious health consequences for consumers, with short-terms effects, such as food poisoning, allergic reactions, or immediate toxicity or even long-term effects such as chronic diseases like cancer, kidney failure, and neurological issues, weakened immunity or nutritional deficiencies.

On the top of that, an issue is in most cases the consumer is unaware of the fact that they are consuming something wrong or by looking at the crop or meat, they cannot make out if it is really fresh within or just a fresh-like.

• Some machine learning based solutions exist to recognize food types, ripeness, or spoilage from a picture of the food item. For example, the Kaggle platform widely hosts publicly available datasets of pictures of food items and various types of associated data typically related to food types, visual characteristics or mechanical properties, that may be used for training and testing machine learning models to recognize food types, ripeness, or spoilage.

However, this does not allow assessing whether the food item is really fresh or has been adulterated to be fresh-like.

The present invention improves the situation.

### SUMMARY

The present disclosure concerns a method of analyzing a food item comprising:
- receiving, from a user device, sensor data relating to the food item including image data and measurement data including odor data;
- determining a food class of the food item from a plurality of food classes from the image data and by using a first trained machine learning model, MLM,
- determining a food quality indicator of the food item based on the measurement data and the determined food class, and
- transmitting the food class and the food quality indicator to the user device for presentation to the user.

Thanks to the invention, a new and inventive solution for quality analysis of a food item is proposed, which combines image analysis with odor detection. Image data are used to predict the food class of the food item and the measurement data the presence of odors and noxious gases that may reveal food adulteration. As a result, the method allows a user of a user device to easily check that a food item not only looks like fresh food, but that it is really of good quality, before consuming it.

For example, the first trained machine learning model is a YOLO ("You Only Look Once"). This is a ML model, available as open source for real-time object detection.

According to an embodiment, the first machine learning model is trained with a first general database comprising a plurality of samples, one of said samples comprising image data of a food item and associated labels comprising at least a food class among the plurality of food classes.

The first general database is typically a publicly available dataset of images of food items, such as Kaggle.com. According to an embodiment, the food class provides a general class or category of the food item, such as "fruit", "vegetable", or a specific class or subcategory, such as "apple", "banana". According to another embodiment, the food class further comprises an indication of a freshness status of the food item of the image data. In this case, the food class is for instance "Fresh apple" or 'Rotten apple".

According to an embodiment, the first machine learning model is further fine-tuned with a first specific training database, comprising a plurality of samples, one sample of said plurality comprising image data of a food item received from the user device and associated labels comprising at least the food class of the food item. This allows to adjust the first MLM to the image data received from the user device and thus to get a more accurate classification of the food item.

According to an embodiment, the food quality indicator is determined by using a second trained machine learning model, MLM, trained with a second training database comprising a plurality of samples, one of said samples comprising measurement data of a food item including odor data, and associated labels including at least a food class among the plurality of food classes and a value of the food quality indicator. In an embodiment, the food quality indicator is determined by using a second trained machine learning model.

For example, the second trained machine learning model is a XGBoost model. This is also an open-source available model, but it is particularly well-suited for tasks such as regression, classification, ranking, time series forecasting, and feature selection, due to its efficiency, scalability, and ability to handle sparse or structured data effectively.

Typically, the food class provided as a label for a food item corresponds to the one used for training and/or retraining the first machine learning model. In an embodiment, the food class further comprises a freshness status of the food item, such as "fresh apple" or "rotten apple". An advantage is to improve a contextual « awareness » of the second machine learning model which may gain a better understanding of a current condition of the food item, leading to more accurate predictions of the food quality indicator.

According to an embodiment, the measurement data further comprises gas measurement data belonging to a group comprising: HCHO data, NH3 data,-H2S data,-NO2 data,CuSO4 data.

These chemical gases are known to be used in food industry for food adulteration. Thus, providing these different types of measurement data allows to detect a variety of gases that may be present in food items and thus to cover a wider range of food adulteration practices.

According to an embodiment, measurement data may also comprise InfraRed data received from an infrared, IR, camera. Indeed, an infrared sensor may reveal various types of information such as temperature of a food item or its surface from a distance by analyzing the emitted infrared radiation. It may also allow measuring the absorption of infrared radiation at different wavelengths and thus deriving a chemical composition of the food item. Presence of certain gases (such as carbon dioxide) may also be detected by measuring their characteristic infrared absorption.

According to an embodiment, the method further comprises the following steps, implemented by a user device:
- collecting the sensor data of the food item, and
- transmitting the captured sensor data to the remote analysis device, through the communication network,
- receiving the food class and the food quality indicator of the food item from the remote analysis device, and
- providing a representation of the food class and the food quality indicator to a user through a human-machine interface of the user device.

In an embodiment, this method is implemented as a software application. Such an application may be lightweight as the determination of the food class and of the food quality indicator, by inference of at least one ML model, is executed by the remote analysis device.

According to an embodiment, capturing the sensor data comprises capturing the image data by taking a picture of the food item with a camera of the user device and capturing the measurement data comprises receiving the measurement data from one or more sensors placed close to the food item, said one or more sensors comprising at least an odor sensor.

In an embodiment, the camera is a visible light camera that captures an RGB picture of the food item.

According to an embodiment, the representation is displayed on a screen of the user device and comprises graphical representations of the image data and the food quality indicator, along with a textual representation of the food class. For instance, the graphical representation of the food quality indicator is a status indicator or color-coded chart or even a heat map to make the result of the assessment understandable at a glance.

In an embodiment, the representation further comprises a graphical representation of a food harmfulness indicator derived from the food quality indicator. An advantage is that it indicates directly to the user if the product is not safe to consume.

In another embodiment, the representation further comprises graphical representations of the measurement data received from the one or more sensors. This allows the user getting more detailed information about the state of the food item.

According to another aspect, a remote analysis device for analyzing quality of a food item comprises a processor operably connected to a memory, said memory comprising a first trained machine learning model, MLM, said remote analysis device being configured to:
- receive, from a user device through a communication network, sensor data of the food item comprising image data and measurement data comprising odor data,
- determine a food class of the food item among a plurality of food classes from the image data and by using the first MLM,
- determine a food quality indicator of the food item based on the measurement data and the determined food class, and
- transmit the food class and the quality food indicator to the user device.

According to another aspect, a user device is configured to cooperate with the aforementioned remote analysis device, said user device comprising a camera configured to capture the image data, a communication interface configured to receive the measurement data from the one or more sensors and a network communication component configured to send the image and measurement data to the remote analysis device through the communication network, to receive the food class and food quality indicator as a response and a human-machine interface configured to provide a representation of the food class and the food quality indicator to a user.

The user device may be any user terminal, such as a smartphone, a tablet, a laptop, etc.

According to yet another aspect, a plug-in sensor device is configured to get plugged to the communication interface of the aforementioned user device, said plug-in sensor device comprising the one or more sensors, among which an odor sensor, and comprising a connector (configured to plug into the communication interface (CI) of the user device and to transmit the measurement data to the user device through said communication interface.

An advantage is that a solid physical connection is established between both devices that makes them move together as one unit. An advantage is that it makes the whole thing very easy to handle to the user.

In an embodiment, the communication interface is a serial communication interface and the plug-in sensor device is powered through the USB connection with the user terminal. An advantage is there is no need to equip the plug-in sensor device with its own power supply. This allows to have it small and light.

In an embodiment, the one or more sensors further comprise one or more gas sensors belonging to a group comprising: a NO2 sensor, a HCHO sensor, an NH3 sensor, a H2S sensor, a CuSO4 sensor.

In an embodiment, the plug-in sensor device further comprises an Infrared, IR, camera.

In yet another embodiment, the plug-in sensor device comprising a case made of an odorless material. This allows to ensure that it does not interfere with the measurements of the one or more sensors unlike plastic materials. For example, the material is acrylic glass, also known as PMMA, which is a lightweight, highly transparent, odorless, and durable material with excellent weather resistance, ease of shaping, and aesthetic versatility.

According to another aspect, a system for quality analysis of a food item, comprises the aforementioned remote analysis device, the aforementioned user device and the aforementioned plug-in sensor device.

According to another aspect, a computer program code comprises program code instructions configured to, when executed by a processor, cause the performance of the steps of the aforementioned method of analyzing a food item.

According to another aspect, a transitory computer-readable medium comprises program instructions stored thereon for causing a computer to perform the aforementioned method of analyzing a food item.

### BRIEF DESCRIPTION OF THE DRAWINGS

Other features, purposes and advantages of the disclosure will become more explicit by means of reading the detailed statement of the non-restrictive embodiments made with reference to the accompanying drawings.
Figure 1 shows a system for analyzing quality of a food item, including a user device, a plug-in sensor device and a remote analysis device, according to an embodiment.
Figure 2 shows an exemplary structure of the plug-in sensor device of Figure 1, according to an embodiment.
Figure 3 is a flowchart of steps of a method of analyzing quality of a food item, implemented by the remote analysis device of Figure 1, according to an embodiment.
Figure 4 schematically illustrates a software implementation of the method on a cloud computing platform, according to an embodiment.
Figure 5 schematically illustrates retraining a first machine learning model to classify image data of the food item into one of a plurality of food classes, according to an embodiment.
Figure 6 schematically illustrates retraining a second machine learning model to determine a food quality indicator for the food item, according to an embodiment.
Figure 7 is a flowchart of a method of analyzing quality of a food item, implemented by the user device and the remote analysis device of Fig. 1, according to another embodiment.
Figures 8A and 8B schematically illustrates examples of representations of the food quality indicator, that are displayed by the user device, according to an embodiment.
Figure 9 shows a communication diagram illustrating the data exchanged within the components of the system for analyzing quality of a food item, according to an embodiment.
Figure 10 schematically illustrates an exemplary hardware structure of a system for analyzing quality of a food item, according to an embodiment.

### DETAILED DESCRIPTION

The present disclosure concerns a computer implemented method of and system of analyzing quality of a food item.

In this disclosure, focus is made on natural and perishable, also called "fresh" food items, that are expected to be minimally or not processed, free from preservatives, and can only be consumed shortly after harvest, production, or preparation. Unlike dry goods, which have a long shelf life, fresh food items typically last only a few days under proper storage conditions, such as refrigeration. Natural and perishable food generally includes fruits and vegetables like apples, tomatoes, or spinach, meat and seafood, or eggs when sold in their natural state.

In this disclosure, it is meant by a food quality indicator an indicator of freshness of the food item that is indicating whether the food item is really fresh or not. The opposite of a fresh product is a spoiled or adulterated product. A food item is spoiled when it has undergone physical, chemical, or microbial changes that make it unsafe, unhealthy, or unpleasant to consume. These changes can affect the food's taste, texture, smell, appearance, or nutritional value. Spoilage can result from natural decay, improper storage, or contamination. A food item may also have been adulterated to be fresh-like.

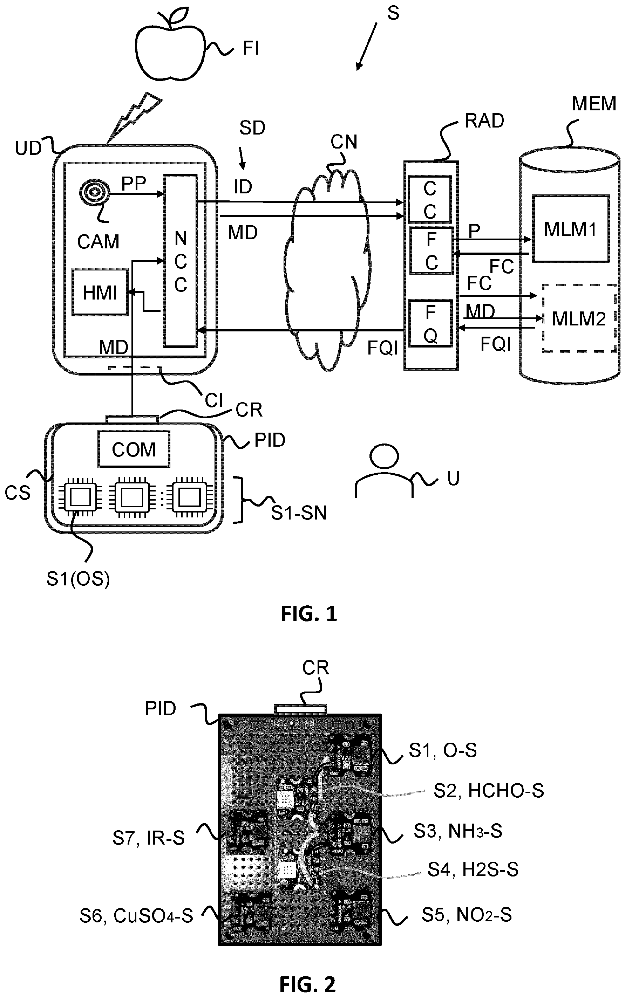

In an embodiment, illustrated by Figure 1, a system S of analyzing quality of a food item FI comprises a remote analysis device RAD, a user device UD and a plug-in sensor device PID. In the example of Figure 1, the food item FI is an apple. In another example, it may be any other fresh food item.

In an embodiment, the remote analysis device RAD comprises a communication component CC configured to receive, from the user device UD through a communication network CN, sensor data SD of the food item comprising image data ID and measurement data MD including odor data, a food class determination component FQ configured to determine a food class FC of the food item FI among a plurality of food classes from the image data and by using a first machine learning model MLM1 and a food quality indicator component FQ configured to determine a food quality indicator FQI of the food item based on the measurement data and the determined food class. The communication component CC is further configured to transmit the food class and the quality food indicator to the user device UD.

In an embodiment, the food quality indicator component FQ is configured to determine a food quality indicator for the food item, by using a second trained machine learning model MLM2.

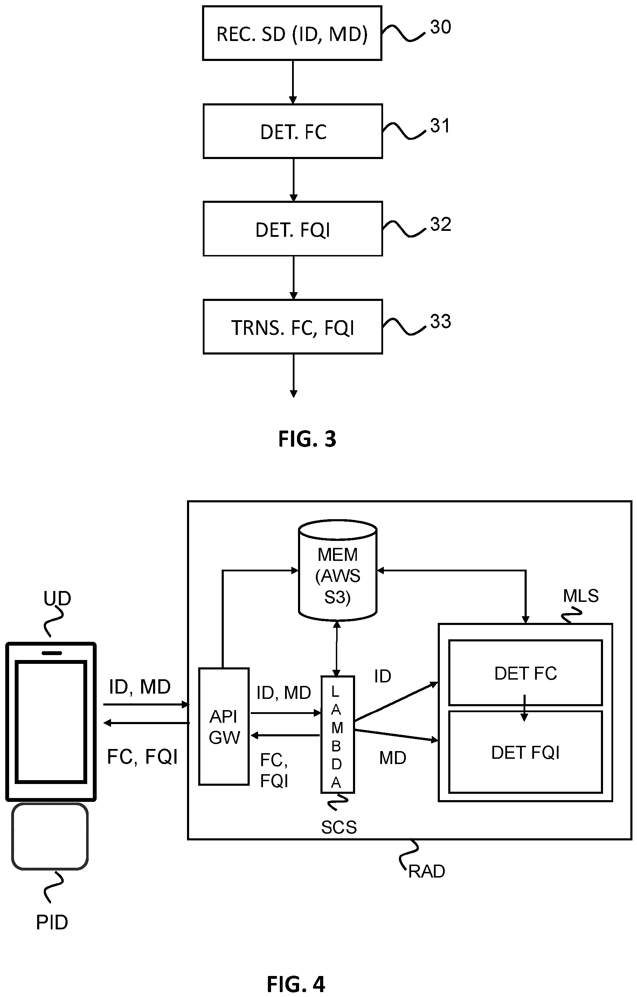

In an embodiment, the remote analysis device RAD is configured to implement a method of analyzing quality of a food item, that will be described hereinafter in relation to Figure 3.

In an embodiment, this method is implemented as a software application.

In another embodiment illustrated by Figure 4, the method is implemented in a distributed manner, for example on one or more services of a cloud computing platform.

In the example of Figure 1, the machine learning model MLM1, and optionally the second machine learning model MLM2, are stored in one or more remote memories or storage devices MEM of the system S. In an alternative, they may be stored in an internal memory of the remote analysis device RAD.

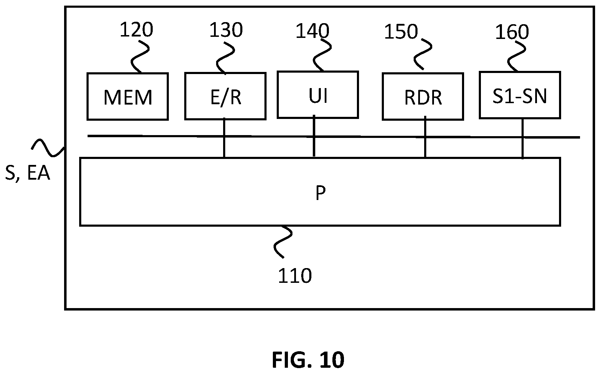

In an embodiment, the remote analysis device RAD is an electronic apparatus EA with the hardware structure of a computer and comprises one or more processors operably connected to one or more memory or storage devices, as will be described hereinafter in relation to Figure 10.

The user device UD of Figure 1 is configured to cooperate with the remote analysis device RAD and comprises a camera CAM configured to collect the image data ID, a communication interface CI configured to receive the measurement data MD from the one or more sensors and a network communication component NCC configured to send the measurement data MD and the image data ID to the remote analysis device RAD through the communication network CN and to receive the food class FC and food quality indicator FQI as a response. The user device UD further comprises a human-machine interface HMI configured to provide a representation of the food class FC and the food quality indicator FQI to a user U.

In an embodiment, the user device UD is a smartphone. In another example, the user device UD may be a tablet or a laptop. These examples are only illustrative and non-limitative. In an embodiment, the camera CAM is a visible light camera that captures an RGB picture of the food item.

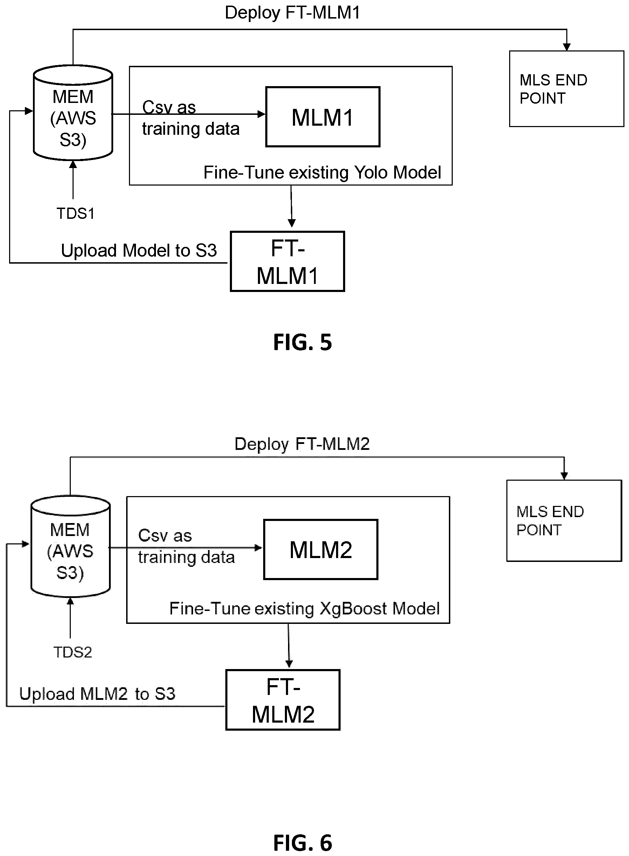

In an embodiment, the user device UD is configured to implement some steps of the method of analyzing quality of a food item, as will be further described in relation to Figure 6:

In an embodiment (not represented), the one or more sensors are integrated into the user device UD.

In an embodiment, these steps of the method are implemented as a software application configured to be executed by the user device UD.

In the embodiment of Figure 1, the plug-in sensor device PID is configured to get plugged to the communication interface CI of the user device UD with a connector CR and comprises the one or more sensors S1-SN, with N a non-zero integer. The one or more sensors S1-SN at least comprise an odor sensor S1(OS). In an embodiment, a DFRobot^{®} MEMS Odor Sensor is used. This is a device designed for detecting and analyzing various odors or Volatile Organic Compounds (VOCs) in the air, which is based on MEMS (Micro-Electro-Mechanical Systems) technology. MEMS (Micro-Electro-Mechanical Systems) Technology refers to the integration of tiny mechanical and electrical components at the microscale, typically fabricated using techniques derived from semiconductor manufacturing. MEMS devices combine sensors, actuators, and electronic circuits on a single chip, enabling them to interact with the physical environment. An advantage of this odor sensor is to be small, efficient, and highly accurate in its readings. Of course, the disclosure is not limited to this example of odor sensor and, in an alternative, an odor sensor based on other technologies, such as an conductive polymer sensor or an electronic nose system (or e-nose) could apply.

In an embodiment, the connector CR in addition to plug into the communication interface CI of the user device UD is further configured to transmit the measurement data MD collected by the one or more sensors S1-SN to the user device UD through the communication interface Cl.

In an embodiment, the communication interface CI is a serial communication interface and the plug-in sensor device PID is powered through the USB connection with the user device UD.

In the embodiment of Figure 1, the plug-in sensor device PID comprises a case CS made of an odorless material.

Referring now to Figure 2, according to an embodiment, the one or more sensors S1-SN are integrated into the plug-in sensor device PID. The first sensor S1 is an odor sensor. S1-SN further comprise some gas sensors. In this example, the second sensor S2 is a HCHO sensor. Formaldehyde (HCHO) in water or Formalin is the simplest colorless aldehyde and has a flammable and pungent odor. It is used for extending storage life. Therefore, presence of HCHO may reveal food alteration.

The third sensor S3 is a NH3 sensor. Ammonia (NH3) is a colorless, poisonous gas with a familiar noxious odor. It occurs in nature, primarily produced by anaerobic decay of plant and animal matter. Sometimes it is combined with HCHO for highly perishable commodity.

The fourth sensor S4 is a H2S sensor. Hydrogen sulfide(H2S) is a colorless chalcogen-hydride gas, which poisonous, corrosive, and flammable. It may be used for meat or fruits preserving.

The fifth sensor S5 is a NO2 sensor. For instance, Nitrate/Nitrite (NO2) is a reddish-brown, pungent, acidic gas that is corrosive and strongly oxidizing. It is used in food industry for meat products to preserve myoglobin binding and pink color.

The sixth sensor S6 is a CuSO4 sensor. A CuSO₄ sensor, also known as a Copper Sulfate sensor, is typically used for detecting and measuring dissolved copper ions (Cu²⁺) in various environments. In particular, CuSo4 is commonly used in pesticides, particularly in fungicides, and serves as a key ingredient in several formulations to control fungal and bacterial diseases in crops.

The seventh sensor S7 is an Infra-Red sensor. Indeed, an infrared (IR) sensor may reveal various types of information such as temperature of a food item or its surface from a distance by analyzing the emitted infrared radiation. It may also allow measuring the absorption of infrared radiation at different wavelengths and thus deriving a chemical composition of the food item. Presence of certain gases (such as carbon dioxide) may also be detected by measuring their characteristic infrared absorption.

Thus, providing these different types of measurement data allows to detect a variety of gases that may be present in food items and thus to cover a wider range of food adulteration practices.

A method of analyzing a food quality item is now described in relation to Figure 3, according to an embodiment. In the following, as an illustrative example, it is implemented by the remote analysis device RAD of Figure 1, but alternatively, any other implementation of the system S may apply.

In a step 30, sensor data SD are received relating to the food item including image data ID and measurement data MD including odor data;

In a step 31 a food class FC of the food item from a plurality of food classes are determined from the image data ID and by using a first trained machine learning model, MLM1. In an embodiment, the plurality of food classes comprises fruit and vegetable names, such as a banana, an apple, a capsicum, a bitter gourd, an orange, a cucumber, etc. In an embodiment, the trained machine learning model is a YOLO (You Only Look Once) machine learning model. YOLO is a known deep learning model designed for real-time object detection, which treats object detection as a single regression problem, predicting bounding boxes and class probabilities directly from an input image in one evaluation. YOLO's architecture combines convolutional neural networks (CNNs) with anchor boxes, enabling efficient detection of multiple objects at different scales within an image. Anchor boxes are predefined rectangular shapes used in object detection models, like YOLO and Faster R-CNN, to predict object locations and dimensions. They serve as templates to approximate the various sizes and aspect ratios of objects in an image. They allow the model to detect objects of varying shapes and sizes and, by providing a starting point for predictions, they simplify the regression task for the model.

In a step 32, a food quality indicator FQI of the food item is determined based on the measurement data MD and the determined food class FC.

In a step 33, the food class FC and the food quality indicator FQI are transmitted to the user device UD for presentation to the user U.

Referring to Figure 4, the method of Figure 3 is implemented as a software in a distributed environment. The illustrative distributed implementation of Figure 4 is based on a cloud computing platform, such as for instance AWS (Amazon Web Services), including compute power, storage options, networking capabilities, machine learning, databases, analytics, etc. Such a cloud computing platform is used by developers to build, deploy, and manage applications and services. In such an environment, the remote analysis device RAD is distributed and leverages the distributed infrastructure of the cloud computing platform. In particular, the memory or storage device MEM of the remote analysis device RAD is structured in the form of an AWS S3 bucket, which allows storing and organizing data in the form of objects, such as files, images, videos, backups, and other types of unstructured data. The objects consist of the data itself, metadata (key-value pairs describing the object), and a unique identifier. In an embodiment, the AWS S3 bucket comprises several folders, one for storing the retrained MLM(s), another for storing the training dataset(s). The AWS S3 bucket may also include a user folder dedicated to store the history of a user using the application with the user device UD.

The remote analysis device RAD further uses an API (Application Programming Interface) gateway API GW, for example an AWS API gateway, that acts as a single entry point for managing, routing, and securing API requests between clients (the user device US) and backend services (the remote analysis device RAD). for receiving the sensor data from the user device UD. An API is a set of rules and protocols that allow two software applications, systems, or components to communicate and exchange data with each other.

The remote analysis device RAD further comprises a serverless computer service SCS, for example an AWS lambda service for running the software application of the method without provisioning or managing servers. The AWS API Gateway provides a way to wrap a Lambda function instead of calling the lambda function directly. Then the application will just invoke the HTTP API endpoint that internally in AWS API Gateway will trigger the lambda function configured.

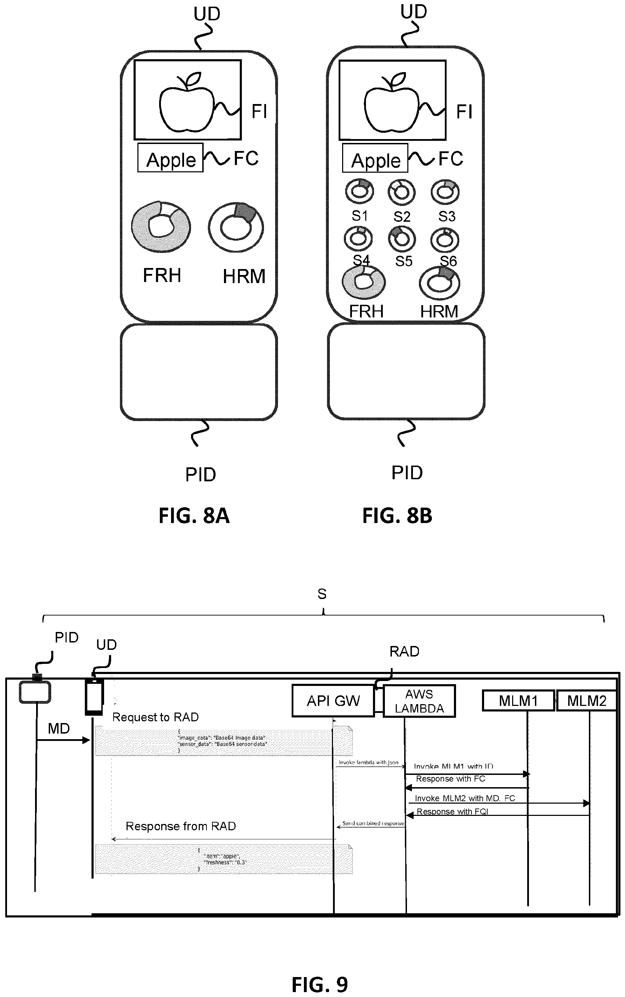

This type of serverless computer service is triggered by events from various other AWS services such as an AWS S3 bucket or external sources like HTTP requests received through the API Gateway. An example of communication messages exchanged between components of the system S when implementing the method of analyzing quality of a food item in a cloud computing infrastructure will be described hereinafter in relation to Figure 9.

In the embodiment of Figure 4, the remote analysis device RAD also exploits a distributed machine learning (ML) service MLS provided by the cloud computing platform to deploy the first machine learning model MLM1 and infer it for determining the food class FC of the food item FI based on the image data ID. An example of such a ML service MLS is AWS SageMaker^{®}.

Referring now to Figure 5, a specific training, or fine-tuning, of the first machine learning model MLM1, for classification of the targeted fresh food items of the present disclosure, is now described in more details. In an embodiment, a first training dataset TDS1 is built by web scrapping from one or public databases comprises images or image data of food items and associated labels (corresponding to food classes). For instance, image data may be uploaded from the Kaggle website at the URL. As a first example, the training database "Fruit360-dataset", available at https://www.kaggle.com/datasets/moltean/fruits/data, provides labelled food classes specifying the specific class of the food item, such as "apple" or even the variety, for instance "golden apple".

As another example, the training data set "Fresh and Rotten Classification", available at https://www.kaggle.com/datasets/swoyam2609/freah-and-stale-classification, provides labelled food classes specifying not only the specific class of the food item, but also a freshness status of the food item, for instance "fresh apple" or "rotten apple".

The built first dataset TDS1 is stored in a memory of the system S, for example a memory MEM of the remote analysis device RAD or, in the embodiment of Figure 4, the AWS S3 storage device. In an embodiment, the images of the first dataset TDS1 are stored in PNG format.

Then the first dataset TDS1 is applied to the first machine learning model MLM1, for example YOLO, A classical supervised approach for retraining a trained machine learning model involves fine-tuning its parameters by applying error backpropagation on a labeled dataset specific to the new application, where the predictions of the machine learning model are compared to the target outputs, and the parameters are adjusted iteratively to minimize the loss function for optimal task performance.

In an embodiment, the first machine-learning model MLM1 is trained using a Jupiter^{®} notebook instance, which is an interactive environment for writing, running, and sharing code.

The fine-tune model FT-MLM1 is further stored in a memory or storage device of the system S, for instance the memory MEM of the remote analysis device RAD or, in the embodiment of Figure 4 in a specific bucket of the AWS S3 storage device. Finally, it is deployed to be inferred by the remote analysis device RAD for determining the food class of a food item, upon receipt of image data ID of this food item from the user device UD. In the embodiment of Figure 3, the fine-tuned model FR-MLM1 is provided to the machine learning service MLS end point for deployment, that is for use in production mode (for real-time classification of food items).

Referring back to Figure 3, in an embodiment, the step 32 of determining a food quality indicator FQI based on the measurement data MD and the determined food class FC is performed by using a second machine learning model MLM2, for example a Xgboost ("extreme Gradient Boosting") model. This model is based on an ensemble of decision trees, where each tree is trained sequentially to improve the performance of the model by correcting errors from previous trees, and it for supervised learning tasks such as regression, classification, and ranking.

Referring now to Figure 6, a specific training, or fine-tuning, of the second machine learning model MLM2, for classification of the targeted fresh food items of the present disclosure, is now described in more details. In an embodiment, a training dataset TDS2 is built from historical measurement data captured by the one or more sensors S1-SN for a plurality of food items, received from the user device UD and stored in a memory of the system S, for instance in the memory MEM of the remote analysis device RAD. This historical measurement data is labelled with the food class FC of the food item.

As an illustrative example, an extract of the second training dataset for apples is provided as table 1 below. The food class of the first column corresponds to the food class provided by the first machine learning model MLM1. In this example, the measurement data MD were captured by the sensors S1-S7 of the plug-in sensor device of Figure 2.

In the example of table 1, this is a food class indicating both the name and a freshness status of the food item. The last column corresponds to a freshness label. In this example, it may only take binary values, 1 for indicating the food item is fresh, 0 otherwise.

**Table 1**

| Food class | S1 : Odor | S2 : HCHO | S3 : NH3 | S4 : H2S | S5 : NO2 | S6 : CuSo4 Sensor | S7 : IR Sensor | Freshness label |
|---|---|---|---|---|---|---|---|---|
| Fresh Apple 1 | 14 | 23 | 20 | 11 | 16 | 15 | 18 | 1 |
| Fresh Apple 2 | 16 | 25 | 21 | 12 | 18 | 17 | 19 | 1 |
| Fresh Apple 3 | 13 | 22 | 19 | 10 | 15 | 14 | 16 | 1 |
| Fresh Apple 4 | 15 | 26 | 22 | 13 | 19 | 20 | 21 | 1 |
| Fresh Apple 5 | 12 | 24 | 18 | 9 | 14 | 13 | 17 | 1 |
| Fresh Apple 6 | 18 | 29 | 25 | 16 | 22 | 21 | 23 | 1 |
| Fresh Apple 7 | 20 | 31 | 27 | 18 | 24 | 23 | 25 | 1 |
| Fresh Apple 8 | 17 | 28 | 26 | 15 | 21 | 19 | 22 | 1 |
| Fresh Apple 9 | 19 | 30 | 24 | 17 | 23 | 25 | 26 | 1 |
| Fresh Apple 10 | 21 | 32 | 28 | 19 | 25 | 27 | 27 | 1 |
| Rotten Apple 1 | 77 | 92 | 72 | 68 | 78 | 80 | 85 | 0 |
| Rotten Apple 2 | 80 | 95 | 75 | 71 | 81 | 82 | 88 | 0 |
| Rotten Apple 3 | 82 | 97 | 78 | 74 | 83 | 85 | 90 | 0 |
| Rotten Apple 4 | 79 | 94 | 73 | 69 | 79 | 81 | 87 | 0 |
| Rotten Apple 5 | 81 | 96 | 76 | 72 | 80 | 83 | 89 | 0 |
| Rotten Apple 6 | 85 | 98 | 80 | 76 | 84 | 86 | 92 | 0 |
| Rotten Apple 7 | 83 | 97 | 79 | 75 | 82 | 84 | 91 | 0 |
| Rotten Apple 8 | 84 | 99 | 81 | 77 | 85 | 88 | 93 | 0 |
| Rotten Apple 9 | 78 | 93 | 71 | 68 | 77 | 79 | 86 | 0 |
| Rotten Apple 10 | 80 | 95 | 74 | 71 | 79 | 82 | 88 | 0 |

In the example of Table 1, all the food items with a "Fresh Apple i" food class are labelled with a freshness label set to 1 and, conversely, all the food items with a "Rotten Apple j" food class are labelled with a freshness label set to 0. However, the whole table may further comprise rows corresponding to food items with a "Fresh" food class and a freshness label set to 0, since the sensor data may allow to reveal that a food item that is classified as fresh from its image is actually not fresh.

Typically, the whole table from which the content of Table 1 is extracted includes 100 or more rows.

The second built dataset TDS2 is stored in a memory of the system S, for example a memory MEM of the remote analysis device RAD or, in the embodiment of Figure 4, an AWS S3 bucket. In an embodiment, the second dataset TDS2 is stored in the CSV (Comma-Separated Values) format, which is a simple file format used to store tabular data, such as spreadsheets or databases, in plain text. Each row in the file represents a data record, and each record is divided into fields separated by a delimiter, typically a comma (,) or any other delimiters like tabs or semicolons format.

Then, the second dataset DS2 is applied to the second machine learning model MLM2. A classical supervised approach for retraining the trained machine learning model is performed.

In a first embodiment, the second machine learning model will learn to output a binary food quality indicator FQI set to 1 when the food item is predicted to be fresh and not adulterated and to 0 when the food item is labelled otherwise.

In another embodiment, the second machine learning model, for instance an XGBoost, will learn to output a real food quality indicator FQI configured to take real value in the range of [0,1]. Indeed; this ML model is natively designed to produce probabilities rather than strict binary labels because of its underlying structure and prediction mechanism. Even when trained with binary labels (0 and 1), it does not directly output these labels but instead learns and outputs probabilities.

The fine-tune model FT-MLM2 is further stored in a memory or storage device of the system S, for instance the memory MEM of the remote analysis device RAD or, in the embodiment of Figure 4 in a specific bucket of the AWS S3 storage device. Finally, it is deployed to be inferred by the remote analysis device RAD for determining the food quality indicator FQI of a food item Fl, upon receipt of measurement data of this food item from the user device UD. In the embodiment of Figure 3, the fine-tuned model FR-MLM2 is provided to the machine learning service MLS end point for deployment, that is for use in production mode (for real-time quality assessment of food items).

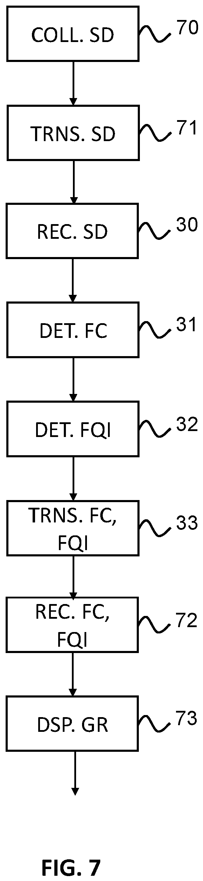

A method of analyzing a food quality item is now described in relation to Figure 7, according to another embodiment. In this example, some steps of this method are implemented by the user device UD of Figure 1, and other steps (from 30 to 33, already described in relation to Figure 3) are implemented by the remote analysis device RAD of Figure 1. However, the present disclosure is not limited to this example, and alternatively, any other implementation of the system S may apply.

In a step 70, sensor data SD related to the food item FI is collected. It includes image data ID from a camera CAM of the user device and measurement data MD captured from one or more sensors. The one or more sensors include an odor sensor, and the measurement data include odor data.

In a step 71, the sensor data SD is transmitted to the remote analysis device, through the communication network CN.

In a step 30 (already described) In a step 20, sensor data SD is received by the remote analysis device RAD from the user device US, relating to the food item FI and including image data ID and measurement data MD including odor data.

In a step 31, a food class FC of the food item from a plurality of food classes is determined from the image data ID and by using a first trained machine learning model, MLM1.

In a step 32 (already described), a food quality indicator FQI of the food item is determined based on the measurement data MD and the determined food class FC.

In a step 33 (already described), the food class FC and the food quality indicator FQI are transmitted to the user device UD for presentation to the user U.

In a step 72, the food class FC and the food quality indicator FQI of the food item are received by the user device UD from the remote analysis device RAD.

In a step 73, a representation of the food class and the food quality indicator are provided to the user U through a human-machine interface HMI of the user device UD.

In an embodiment, a graphical representation GR is displayed on the screen of the user device UD comprising a textual indication corresponding the food class FC or, when the food class also comprises an indication of freshness status, only the food name, of the food item and a color-coded chart of the food quality indicator FQI.

Referring to Figure 8A, the representation further comprises a representation of the image data ID of the food item Fl, here an apple. In the example of Figure 7A, the food quality indicator FQI is displayed as a freshness FRH ring, the fresher the food item, the fuller the ring. For instance, a color legend is used, with green indicating the best and red indicating the worst. In an embodiment, the FQI values are ranging between 0 and 1 (or 0 to 100%). For representation purposes, they are for instance segregated into 4 categories. <25% - Red, <50% indicating the food item is not fresh- Orange, ≥50% and <75% indicating the food item is not semi-fresh - Yellow, ≥75% and <100% - green, indicating the food item is fresh.. An advantage is to make the result of the assessment understandable at a glance.

In an embodiment, another color chart representative of an indicator of harmfulness HRM is also displayed, the more harmful the food item, the fuller the ring. For instance, the associated color is red. This indicator of harmfulness HRM is for instance derived from the food quality indicator, such as FQI+ HRM = 1.

An advantage of displaying such a food harmfulness indicator is that it indicates directly to the user if the food item is not safe to consume.

Referring to Figure 8B, according to another embodiment, the representation further comprises graphical representations of the measurement data received from each of the one or more sensors S1-SN. For instance, they are also represented by rings, whose filling rate increases with the quantity of gas detected. This representation of the measurement data MD is for instance generated by the user device UD. This allows the user getting more detailed information about the state of the food item.

In an embodiment, these steps 70-73 of the method are implemented as a software application configured to be executed by the user device UD. Such an application is lightweight as the determination of the food class and of the food quality indicator, by inference of at least one ML model, is executed by the remote analysis device, not by at the user device. Therefore, the software application only needs transmit the sensor data as a request and receive the determined food class and food quality indicator as a response.

Referring to Figure 9, an example of communication between components of the system S to implement the above-described method of analyzing a food item is now described according to an embodiment. In this example, the steps 30-33 are executed on a cloud computing platform, as described above in relation to Figure 4.

For instance, the software application is launched by the user U of the user device UD who wants to get feedback on quality of a food item before consuming it. A picture is taken of the food item by the software application using the camera CAM of the user device UD.

The software application is also configured to trigger the capture of measurement data MD by the plurality of sensors S1-SN. In an embodiment, the software application has a scan functionality that allows the user to a picture using the camera CAM and at the same time, trigger the capture of measurement data MD by the sensors. Both ID and MD data are then sent for analysis to the backend (RAD).

In the example, the plurality of sensors S1-SN is integrated into the plug-in sensor device PID of Figure 2.

Once received, the measurement data MD are transmitted along with the image data to the remote analysis device RAD in a HTTP request through the API Gateway. The API gateway, upon receipt of the HTTP request is configured to invoke the AWS lambda function "AWS_Lambda" by providing input formatted with a JSON (JavaScript Object Notation) payload as an event that is transmitted to the cloud computing platform. JSON is a lightweight data-interchange format that is easy for humans to read and write and for machines to parse and generate. The lambda function (AWS LAMBDA) is configured to process this input and execute custom logic, returning results based on the provided input data, here the sensor data. With the present method, executing custom logic means invoking by the lambda function execution of step 31 of determining the food class of the food item based on the image data ID by using the first machine learning model MLM1, for instance with a ML service such as AWS SageMaker^{®}. A first request comprising the image data ID is sent to MLM1. Upon receipt of a first response from the MLM1 including the food class FC, the lambda function is configured to invoke execution of step 32 of determining the food quality indicator FQ. In the example of Figure 9, this determination is performed by using the second machine learning model MLM2, deployed by a ML service of the cloud computing platform, such as AWS SageMaker^{®}. A second request comprising the determined food class FC along with the measurement data MD is sent to the second machine learning model MLM2. In an alternative, as illustrated by Figure 4, the food class FC is directly provided by the MLM1 to the MLM2.

A second response comprising the food quality indicator FQI is received by the lambda function, which is configured to combine the first and second responses in a single response to the initial invocation of the lambda function, that is sent to the user device UD through the API gateway. In the example of Figure 9, the response includes the food class "apple" and a value of food quality indicator, or freshness indicator, equal to 0.3, indicating that the food item is considered as not fresh.

Each function, block, step described herein may be implemented in hardware, software, firmware, middleware, microcode or any suitable combination thereof. If they are implemented in software, the functions or blocks of the block diagrams and flowcharts can be implemented by computer program instructions/software codes, which can be stored or transmitted on a computer-readable medium, or loaded onto a general-purpose computer, special-purpose computer or other programmable processing device and/or system, so that the computer program instructions or software codes running on the computer or other programmable processing device create the means to implement the functions described in the present description.

In an embodiment, the computer program instructions or codebase of each function, block, step are containerized and deployed in a distributed computing architecture, for example a serverless cloud platform.

Figure 10 shows an example of the hardware structure of a system S of analyzing a food item, according to another embodiment. In this example, the system S is integrated into an electronic apparatus EA comprising at least one processor 110 and at least one memory 120. The electronic apparatus EA may also comprise one or more communication interfaces. In this example, the electronic apparatus EA comprises network interfaces 130 (for example, network interfaces for wired/wireless network access, including an Ethernet interface, a WIFI interface, etc.) connected to the processor 110 and configured to communicate via one or more wired/wireless communication links and user interfaces 140 (for example, a keyboard, a mouse, a display screen, etc.) connected to the processor. The electronic apparatus EA may also comprise one or more media players 150 for reading a computer-readable storage medium (for example, a digital storage disk (CD-ROM, DVD, Blue Ray, etc.), a USB stick, etc.). The electronic apparatus EA also comprises a plurality of sensors 160 including a camera and an odor sensor, configured to capture measurement data related to a food item. The processor 110 is connected to each of the other aforementioned components in order to control the operation thereof.

The memory 1120 may comprise a random-access memory (RAM), cache memory, non-volatile memory, backup memory (for example, programmable or flash memories), read-only memory (ROM), a hard disk drive (HDD), a solid-state drive (SSD) or any combination thereof. The ROM of the memory 120 can be configured to store, inter alia, an operating system of the electronic apparatus EA and/or one or more computer program codes of one or more software applications. The RAM of the memory 120 can be used by the processor 110 for temporary data storage.

The processor 110 can be configured to store, read, load, execute and/or else process instructions stored in a computer-readable storage medium and/or in the memory 120 so that, when the instructions are executed by the processor, one or more or all of the steps of the methods disclosed herein are performed. Means implementing a function or set of functions may correspond in this document to a software component, a hardware component or even a combination of hardware and/or software components, capable of implementing the function or set of functions, as described below for the means related.

The present description also relates to an information storage medium readable by a data processor, and comprising instructions of a program as mentioned above. The information storage medium can be any hardware means, entity or apparatus, capable of storing the instructions of the aforementioned computer program. Usable program storage media include ROM or RAM, magnetic storage media such as magnetic disks and tapes, hard disks or optically readable digital data storage media, or any combination thereof.

In some cases, the computer-readable storage medium is non-transitory. In other cases, the information storage medium may be a transient medium (for example, a carrier wave) for transmitting a signal (electromagnetic, electrical, radio or optical signal) containing program instructions. This signal can be routed via a suitable wired or wireless transmission means: electrical or optical cable, radio or infrared link, or by other means.

## Claims

1. A method of analyzing a food item comprising:
- receiving (30), from a user device (UD), sensor data (SD) relating to the food item including image data (ID) and measurement data (MD) including odor data;
- determining (31) a food class (FC) of the food item from a plurality of food classes from the image data and by using a first trained machine learning model (MLM1),
- determining (32) a food quality indicator (FQI) of the food item based on the measurement data and the determined food class, and
- transmitting (33) the food class and the food quality indicator to the user device (UD)for presentation to the user.

2. The method according to claim 1, wherein the first machine learning model is trained with a first general database comprising a plurality of samples, one of said samples comprising image data of a food item and associated labels comprising at least a food class among the plurality of food classes.

3. The method according to the preceding claim, wherein the first machine learning model is further fine-tuned with a first specific training database (TDS1), comprising a plurality of samples, one sample of said plurality comprising image data of a food item received from the user device and associated labels comprising at least the food class of the food item.

4. The method according to anyone of the preceding claims, wherein the food quality indicator is determined by using a second trained machine learning model (MLM2), trained with a second training database (TDS2) comprising a plurality of samples, one of said samples comprising measurement data of a food item including odor data, and associated labels including at least a food class among the plurality of food classes and a value of the food quality indicator.

5. The method according to anyone of the preceding claims, wherein the measurement data further comprises gas measurement data belonging to a group comprising:
- HCHO data,
- NH3 data,
- H2S data,
- NO2 data,
- CuSO4 data.

6. The method according to anyone of the preceding claims, further comprising the following steps, implemented by a user device (UD):
- collecting (70) the sensor data of the food item, and
- transmitting (71) the captured sensor data to the remote analysis device, through the communication network,
- receiving (72) the food class and the food quality indicator of the food item from the remote analysis device, and
- providing (73) a representation of the food class and the food quality indicator to a user through a human-machine interface of the user device.

7. The method according to the preceding claim, wherein capturing the sensor data comprising capturing the image data by taking a picture of the food item with a camera of the user device and capturing the measurement data comprises receiving the measurement data from one or more sensors placed close to the food item, said one or more sensors comprising at least an odor sensor.

8. The method according to anyone of the claims 6 to 7, wherein the representation is displayed on a screen of the user device and comprises graphical representations (GR) of the image data and the food quality indicator, along with a textual representation of the food class.

9. A remote analysis device (RAD) for analyzing quality of a food item, comprising a processor operably connected to a memory, said memory comprising a first trained machine learning model (MLM1), said remote analysis device being configured to:
- receive, from a user device through a communication network, sensor data of the food item comprising image data and measurement data comprising odor data,
- determine a food class of the food item from a plurality of food classes from the image data and by using the first machine learning model (MLM1),
- determine a food quality indicator of the food item based on the measurement data and the determined food class,
- transmit the food class and the quality food indicator to the user device (UD).

10. A user device (UD) configured to cooperate with the remote analysis device (RAD) according to the preceding claim, said user device comprising a camera (CAM) configured to capture the image data (ID), a communication interface (CI) configured to receive the measurement data (MD) from the one or more sensors (S1-SN) and a network communication component (NCC) configured to send the image and measurement data to the remote analysis device (RAD) through the communication network (CN), to receive the food class (FC) and food quality indicator (FQI) as a response and a human-machine interface (HMI) configured to provide a representation of the food class and the food quality indicator to a user.

11. A plug-in sensor device (PID) configured to get plugged to the communication interface (CI) of the user device (UD) according to claim 10, said plug-in sensor device comprising the one or more sensors (S1-S7), among which an odor sensor (O-S, S1), and comprising a connector (CR) configured to plug into the communication interface (CI) of the user device (UD) and to transmit the measurement data (MD) to the user device (UD) through said communication interface (CI).

12. The plug-in sensor device according to the previous claim, wherein the one or more sensors further comprise one or more gas sensors belonging to a group comprising:
- a NO2 sensor,
- a HCHO sensor,
- an NH3 sensor,
- a H2S sensor,
- a CuSO4 sensor.

13. A system (S) for quality analysis of a food item, comprising a remote analysis device (RAD) according to claim 9, a user device (UD) according to claim 10 and a plug-in sensor device (PID) according to anyone of claims 11 and 12.

14. A computer program code, comprising program code instructions configured to, when executed by a processor, cause the performance of the steps of the method of any of claims 1 to 8.

15. A transitory computer-readable medium comprises program instructions stored thereon for causing a computer to perform a method according to any one of claims 1 to 8.
